# EUROPEAN PATENT APPLICATION

(11) **EP 1 579 824 A2**
(43) Date of publication of application: **28.09.2005**
(21) Application number: 04077765.8
(22) Date of filing: 28.06.2000
(51) Int. Cl.: A61F 2/06

(54) **Variable thickness stent and method of manufacture thereof**

(30) Priority: 30.06.1999 US 343962
(62) Divisional of application: 00945038.8
(71) Applicant: ADVANCED CARDIOVASCULAR SYSTEMS, INC., California 95054 (US)
(72) Inventor: Padilla, Orlando M., Laguna Niguel California 92677 (US); Mirizzi, Michael S., Santa Clara California 95054 (US); Wynne, Robert L. c/o Advanced Cardiovascular, Santa Clara, California 95054-8167 (US); Callol, Joseph R., San Francisco California 94114 (US); Kitchen, Tim L., San Francisco California 94110 (US)
(74) Representative: McLeish, Nicholas Alistair Maxwell

(57) **Abstract**

The invention is directed to a stent having varying thickness, with the thickness selected based at least in part on the flexing characteristics of the particular portion of the stent. The stent is formed with a pattern having flexing and stable portions, with the flexing portions contributing to stent flexibility during stent positioning and deployment. One or more flexing portions are provided with a nominal thickness, and at least some stable portions are provided with a greater-than-nominal thickness. The resulting stent has excellent flexibility, strength, and radiopacity characteristics. The stent may be formed using various approaches, including using a tubular member, flat sheet, or wire.

## Description

### BACKGROUND OF THE INVENTION

This invention relates to devices for the treatment of heart disease and particularly to endo-arterial prostheses, which are commonly called stents. More particularly, the invention relates to stents having variable thickness.

Several interventional treatment modalities are presently used for heart disease, including balloon and laser angioplasty, atherectomy, and by-pass surgery. In typical coronary balloon angioplasty procedures, a guiding catheter having a distal tip is percutaneously introduced through the femoral artery into the cardiovascular system of a patient using a conventional Seldinger technique and advanced within the cardiovascular system until the distal tip of the guiding catheter is seated in the ostium of a coronary artery. A guidewire is positioned within an inner lumen of a dilatation catheter and then both are advanced through the guiding catheter to the distal end thereof. The guidewire is first advanced out of the distal end of the guiding catheter into the patient's coronary vasculature until the distal end of the guidewire crosses a lesion to be dilated, then the dilatation catheter having an inflatable balloon on the distal portion thereof is advanced into the patient's coronary anatomy over the previously introduced guidewire until the balloon of the dilatation catheter is properly positioned across the lesion. Once in position across the lesion, the balloon is inflated to compress the plaque of the lesion against the inside of the artery wall and to otherwise expand the inner lumen of the artery. The balloon is then deflated so that blood flow can be resumed through the dilated artery and the dilatation catheter can be removed therefrom. Further details of dilatation catheters, guidewires, and devices associated therewith for angioplasty procedures can be found in U.S. Pat. No. 4,323,071 (Simpson-Robert); U.S. Pat. No. 4,439,185 (Lindquist); U.S. Pat. No. 4,516,972 (Samson); U.S. Pat. No. 4,538,622 (Samson, et al.); U.S. Pat. No. 4,554,929 (Samson, et al.); U.S. Pat. No. 4,616,652 (Simpson); U.S. Pat. No. 4,638,805 (Powell); U.S. Pat. No. 4,748,982 (Horzewski, et al.); U.S. Pat. No. 5,507,768 (Lau, et al.); U.S. Pat. No. 5,451,233 (Yock); and U.S. Pat. No. 5,458,651 (Klemm, et al.), which are hereby incorporated herein in their entirety by reference thereto.

One problem that can occur during balloon angioplasty procedures is the formation of intimal flaps which can collapse and occlude the artery when the balloon is deflated at the end of the angioplasty procedure. Another problem characteristic of balloon angioplasty procedures is the large number of patients who are subject to restenosis in the treated artery. In the case of restenosis, the treated artery may again be subjected to balloon angioplasty or to other treatments such as by-pass surgery, if additional balloon angioplasty procedures are not warranted. However, in the event of a partial or total occlusion of a coronary artery by the collapse of a dissected arterial lining after the balloon is deflated, the patient may require immediate medical attention, particularly in the coronary arteries.

A focus of recent development work in the treatment of heart disease has been directed to endoprosthetic devices called stents. Stents are generally cylindrically shaped intravascular devices which are placed within an artery to hold it open. The device can be used to reduce the likelihood of restenosis and to maintain the patency of a blood vessel immediately after intravascular treatments. In some circumstances, they can also be used as the primary treatment device where they are expanded to dilate a stenosis and then left in place. Further details of stents can be found in U.S. Patent No. 3,868,956 (Alfidi et al.); U.S. Patent No. 4,512,338 (Balko et al.); U.S. Patent No. 4,553,545 (Maass et al.); U.S. Patent No. 4,733,665 (Palmaz); U.S. Patent No. 4,762,128 (Rosenbluth); U.S. Patent No. 4,800,882 (Gianturco); U.S. Patent No. 4,856,516 (Hillstead); U.S. Patent No. 4,886,062 (Wiktor); U.S. Patent No. 5,421,955 (Lau); and U.S. Patent No. 5,569,295 (Lam), which are hereby incorporated herein in their entirety by reference thereto.

One method and system developed for delivering stents to desired locations within the patient's body lumen involves crimping a stent about an expandable member, such as a balloon on the distal end of a catheter, advancing the catheter through the patient's vascular system until the stent is in the desired location within a blood vessel, and then inflating the expandable member on the catheter to expand the stent within the blood vessel. The expandable member is then deflated and the catheter withdrawn, leaving the expanded stent within the blood vessel, holding open the passageway thereof.

Advancing the stent through a patient's vasculature, which can involve traversing sharp bends and other obstacles, may require the stent to be highly flexible. Stent flexibility also permits the stent to be deployed in and conform to a tortuous section of a patient's vasculature. Additionally, visualizing the stent with a fluoroscope, which is currently the most widely used method of stent visualization during stent deployment, requires a stent with good radiopacity.

Different methods have been attempted to give stents high flexibility and radiopacity. By making stents out of relatively thin material, flexibility can be increased. However, the use of thin material can reduce the radiopacity of the stent, which can make it difficult for a user to visualize the stent. Conversely, the use of thicker material, which can promote radiopacity, can reduce stent flexibility, which can impair the deliverability of the stent.

An early attempt at achieving a flexible stent with good radiopacity characteristics involved providing a stent of a base material with good flexibility and strength but relatively low radiopacity, and then adding a thin layer of a highly-radiopaque material, such as gold, to the stent. This approach, which required the use of two separate materials, involved a relatively complicated process in applying the radiopaque material to the stent. Additionally, the use of multiple materials can complicate use and deployment of the stent, particularly where the different materials have different material characteristics, such as different strengths, different biocompatibility, or different responses to temperature changes.

Another approach was to provide a stent with substantially thicker portions at each end. Such an approach provided a stent with highly radiopaque ends, so that a physician could easily view the stent ends during stent delivery.

What has been needed and heretofore unavailable is an improved means of providing a stent with high flexibility, strength, and radiopacity. The present invention satisfies this need.

### SUMMARY OF THE INVENTION

The invention is directed to a stent having desirable flexibility, strength, and radiopacity characteristics. In particular, the stent of the invention has varying thickness, with a smaller nominal thickness that promotes flexibility, and a larger thickness that promotes radiopacity.

In many expandable stent designs, there are portions of the stent that flex or otherwise deform during bending of the stent. Similarly, there are also portions of the stent that remain stable (i.e., remain largely undeformed) during bending.

For example, when a stent is introduced into a coronary artery, the stent must longitudinally bend in order to navigate curves in the patient's vasculature. When the stent is bent while passing through bends in an artery, some portions of the stent will flex. Other portions, however, will remain largely undeformed, even when the stent as a whole is bent through extremely tight turns. Thus, the stent has flexing portions and stable (i.e., undeformed) portions.

The flexibility of a stent is largely derived from the portions of the stent that flex, without substantial assistance from the stable portions. Stable portions are those portions that, due to the overall stent design, are not subject to sufficient loads to cause substantial bending or flexing of the particular portion. Some of these so-called "stable" portions might, if cut from the stent and subjected to a sufficient load, deform substantially. However, when such a portion is part of an integral stent, it is not subject to such loads, and accordingly does not substantially bend or flex. The stable portions provide strength, but do not appreciably flex or otherwise deform. Accordingly, increasing the thickness of these stable portions may not materially impact overall stent flexibility or ease of deployment.

When a stent is expanded from its delivery diameter to its deployed diameter, certain portions of the stent will deform, while others remain undeformed. Depending on the particular stent design, the areas that flex during stent bending may coincide with the areas that deform during stent expansion.

Some other stent designs have reduced the density of the stent pattern to promote flexibility. For example, in a stent design having convoluted ring-like elements connected by links, reduction in density can be achieved by reducing the number of convolutions per ring. Reducing stent pattern density can reduce the uniformity of the stent's expansion, and can also reduce the coverage of the arterial wall by the deployed stent. Such arterial wall coverage is also known as scaffolding.

In order to balance the need for increased stent thickness, which is driven by radiopacity requirements, with the need for decreased stent thickness, which is driven by flexibility/deployment requirements, a preferred embodiment of the current invention provides a stent having thinner areas that promote flexibility, with greater thickness in selected areas in order to promote stent radiopacity/visibility. By coinciding the thicker areas of the material with the stable areas of the stent, overall stent flexibility/expansion is not impacted by the thicker, stable areas. The approach can be applied to stents having high-density stent patterns that, if constructed of consistent thickness, might have insufficient flexibility. Such high-density stent patterns can thus be used, in conjunction with the variable thicknesses of the invention, to construct highly flexible, radiopaque stents having high-density stent patterns that promote good arterial wall coverage (i.e., stent scaffolding) of the deployed stent. The approach can also be used to promote uniform stent expansion.

The stent of the present invention preferably includes an elongated tubular body having a first end section, a second end section, and a central section therebetween. In a preferred embodiment, the elongated tubular body has an open lattice structure or weave that is adapted for radial expansion from a first, compressed (delivery) diameter, to a second expanded (deployed) diameter which approximates the inner diameter of the body lumen in which the stent is to be implanted.

In one embodiment of the invention, additional material is added to stable portions of the stent, thereby increasing the thickness (and radiopacity) of those portions. In general, increasing thickness of a structure reduces its flexibility. However, because the stable portions of the stent did not flex to begin with, increasing their thickness will not materially impact overall stent flexibility. The additional material is preferably the same as the material from which the rest of the stent is formed, so that the stent overall is formed from a single material.

In another embodiment of the invention, the process of stent manufacture starts with a stent of greater-than-nominal thickness. The thickness of selected areas of the stent is reduced to a preferred nominal thickness. Such an embodiment may include reducing the thickness of substantially all flexing portions of the stent down to a nominal thickness conducive to flexibility, in order to assure that the stent is flexible during delivery. Thicker material is maintained on at least some part of the stable portions, which provide good radiopacity characteristics. Thus, the stent will have good flexibility and good radiopacity, so that the stent has good deliverability characteristics and also allows a surgeon to easily view the stent with a fluoroscope.

In one embodiment, the stent of the invention generally includes a plurality of radially expandable cylindrical elements which are relatively independent in their ability to expand and to flex relative to one another. The individual radially expanded cylindrical elements of the stent are dimensioned so as to be longitudinally shorter than their own diameters. Interconnecting elements or struts extending between adjacent cylindrical elements provide increased stability and are preferably positioned to prevent warping of the stent upon the expansion thereof. The resulting stent structure is an open lattice having a series of radially expandable cylindrical elements which are spaced longitudinally close enough so that small dissections in the wall of a body lumen may be pressed back into position against the lumen wall, but not so close as to compromise the longitudinal flexibility of the stent. The individual cylindrical elements cumulatively provide a stent which is flexible along its longitudinal axis, but where flexing portions of the stent have a nominal thickness for maximum flexibility and strength, and at least some stable portions of the stent have a greater-than-nominal thickness for enhanced radiopacity and strength.

The stent embodying features of the invention can be readily delivered to the desired body lumen location by mounting it on an expandable member of a delivery catheter, such as a balloon catheter, and passing the catheter-stent assembly through the body lumen to the implant site. A variety of means for securing the stent to the expandable member on the catheter for delivery to the desired location are available. It is presently preferred to compress the stent onto the balloon. Other means to secure the stent to the balloon include applying heat and/or pressure to the balloon/stent assembly, providing a retractable sheath over the stent, having ridges or collars on the inflatable member to restrain lateral movement of the stent, and/or using bioresorbable temporary adhesives to hold the stent on the balloon. Various delivery modes may also be used, depending on the particular application and stent construction. For example, a self-expanding stent, such as a stent formed from nitinol (NiTi), might be delivered inside a tubular catheter, and then ejected from the tubular catheter to self-expand at the desired deployment site.

A presently preferred structure for the expandable cylindrical elements which form the stents of the present invention are generally circumferential undulating patterns, e.g., a generally serpentine pattern. The open reticulated structure of the stent provides excellent coverage of the arterial wall to prevent restenosis, while allowing for the perfusion of blood over a large portion of the arterial wall which can improve the healing and repair of a damaged arterial lining.

The stent can be formed from a variety of materials, including metals or polymers. Biocompatible materials are generally preferred, depending on the application. Stainless steel, which is currently in wide use in stents, is one example of a metal that could be used in the invention. So-called self-expanding materials, such as nitinol, could also be used.

The invention results in a stent having desirable flexibility, strength, and radiopacity characteristics. The stent is relatively easy to manufacture, and can be formed from a single material.

Other features and advantages of the invention will become apparent from the following detailed description, taken in conjunction with the accompanying drawings, which illustrate, by way of example, the features of the invention.

### BRIEF DESCRIPTION OF THE DRAWINGS

FIGURE 1 is an elevational view, partially in section, of a stent embodying features of an embodiment of the invention which is mounted on a delivery catheter and disposed within an artery.
FIG. 2 is an elevational view, partially in section, similar to that shown in FIG. 1, wherein the stent is expanded within an artery, pressing the lining against the arterial wall.
FIG. 3 is an elevational view, partially in section, showing the expanded stent within the artery after withdrawal of the delivery catheter.
FIG. 4 depicts a stent pattern from the stent embodiment of FIG. 1.
FIGS. 5a and 5b depict a close-up of a portion of the stent pattern from FIG. 4.
FIG. 6a depicts in perspective with partial cross-section a close-up portion of a stent.
FIG. 6b depicts in perspective with partial cross-section a close-up portion of a stent in accordance with an embodiment of the invention.
FIG. 6c depicts in perspective with partial cross-section a close-up portion of a stent in accordance with a further embodiment of the invention.
FIG. 7 depicts a side view, in cross-section, of a portion of a stent in accordance with an embodiment of the invention.
FIGS. 8a and 8b depict in side view a stent being manufactured in accordance with an embodiment of the invention.
FIG. 9a depicts a tubular rough stent form in accordance with en embodiment of the invention.
FIG. 9b depicts a stent pattern in accordance with an embodiment of the invention.
FIGS. 10a and 10b depict, in side view, a stent being manufactured in accordance with an embodiment of the invention.
FIG. 10c depicts a side view, in cross-section, of a stent in accordance with an embodiment of the invention.
FIG. 11 depicts an end view of a stent being manufactured in accordance with an embodiment of the invention.
FIGS. 12a and 12b depict a side view, in cross-section, of a portion of a stent in accordance with various embodiments of the invention.
FIGS. 13a, 13b, 13c, and 13d depict a side view, in cross-section, of a portion of a stent in accordance with various embodiments of the invention.
FIGS. 14a, 14b, and 14c depict perspective views of a stent being manufactured in accordance with an embodiment of the invention.
FIGS. 15a and 15b depict a perspective view of stents in accordance with various embodiments of the invention.
FIGS. 16a, 16b, and 16c depict a side view, in cross-section, of a portion of a stent in accordance with various embodiments of the invention.
FIGS. 17a and 17b depict a side view, in cross-section, of portions of a stent mounted on a balloon in accordance with an embodiment of the invention.
FIGS. 18a, 18b, and 18c depict a side view, in cross-section, of a portion of a stent in accordance with various embodiments of the invention.
FIG. 19a depicts a stent pattern in accordance with an embodiment of the invention.
FIG. 19b depicts a tubular rough stent form in accordance with an embodiment of the invention.
FIGS. 20a and 20b depict a side view, in cross-section, of a portion of a stent mounted on a balloon in accordance with an embodiment of the invention.
FIGS. 21a and 21b depict a side view, in cross-section, of a portion of a stent mounted on a balloon in accordance with an embodiment of the invention.

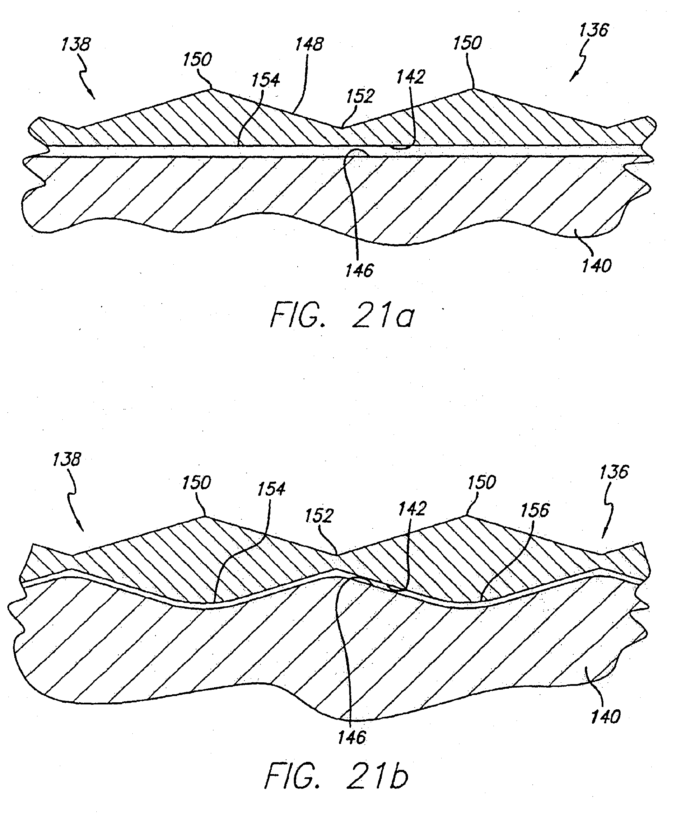

FIGS. 20a and 20b depict a side view, in cross-section, of a stent in accordance with an embodiment of the invention.

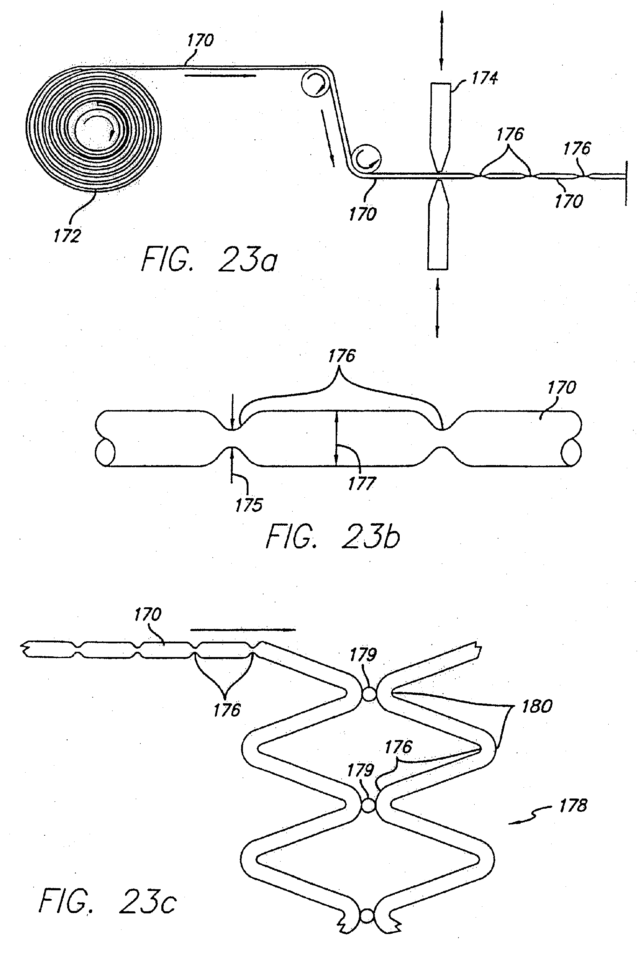

FIG. 23a, 23b, and 23c depict a side view of a wire being formed into a stent in accordance with an embodiment of the invention.

### DETAILED DESCRIPTION OF THE PREFERRED EMBODIMENTS

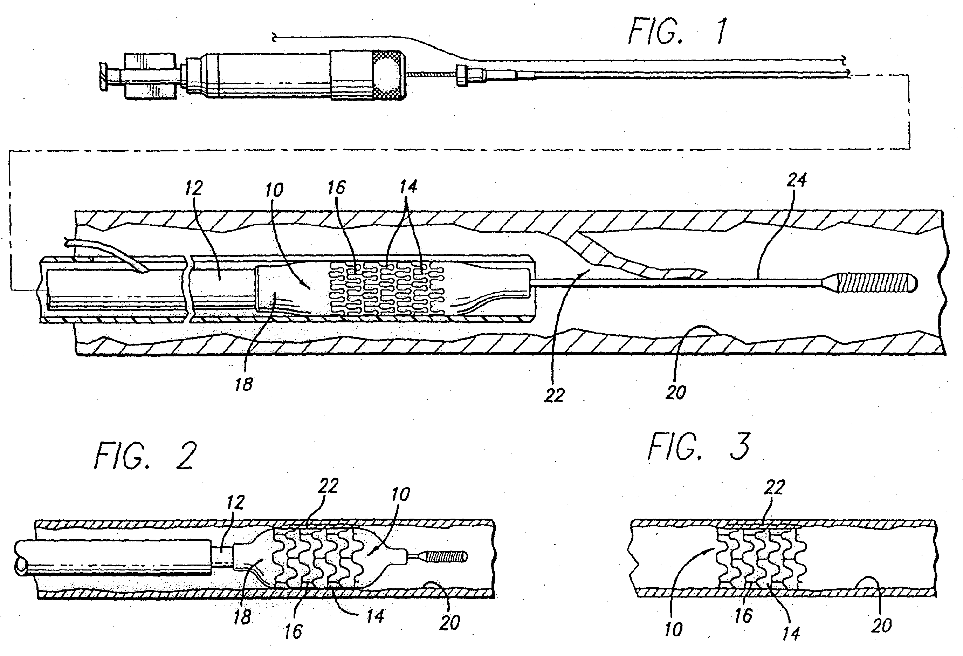

The present invention is depicted in FIGS. 1-23 for use in various body lumens and procedures, including use in dilated arteries during balloon angioplasties. However, the present invention is not limited to use in blood vessels or angioplasties, but can be used in other body lumens and procedures, including treatment of urinary, digestive, or bile ducts.

The stent of the preferred embodiment is generally delivered intraluminally using a conventional balloon catheter as is known in the art. The stent is used primarily to ensure the patency of the body lumen in which it is implanted. For example, the stent may be implanted in the coronary arteries after an angioplasty procedure to reinforce the artery against recoil or to tack up a dissection in the arterial wall. The stent is useful for implanting in other body lumens, such as the carotid arteries, illiacs, cerebral vasculature, and other peripheral veins and arteries.

FIG. 1 illustrates a stent 10, incorporating features of the invention, which is mounted on a delivery catheter 12. The stent 10 in the embodiment depicted comprises a plurality of radially expandable cylindrical elements 14 disposed generally coaxially and interconnected by links 16 disposed between adjacent cylindrical elements 14. The delivery catheter 12 has an expandable portion or balloon 18 for expanding the stent 10 within coronary artery 20. The artery 20, as shown in FIG. 1, has a dissected lining 22 which has occluded a portion of the arterial passageway.

The delivery catheter 12 onto which the stent 10 is mounted is essentially the same as a conventional balloon dilatation catheter for angioplasty procedures. The balloon 18 may be formed of suitable materials such as polyethylene, polyethylene terephthalate, polyvinyl chloride, nylon and ionomers such as Surlyn® manufactured by the Polymer Products Division of the Du Pont Company. Other polymers may also be used.

In order for the stent 10 to remain in place on the balloon 18 during delivery to the site of the damage within the artery 20, the stent 10 is compressed onto the balloon. Other means for securing the stent 10 onto the balloon 18 may also be used, such as providing a covering sheath over the stent, or providing collars or ridges on the ends of the working portion, i.e., the cylindrical portion, of the balloon. Thermal and/or pressure processes can also be used to secure the stent to the balloon.

The delivery of the stent 10 is accomplished in the following manner. The stent 10 is first mounted onto the inflatable balloon 18 on the distal extremity of the delivery catheter 12. The balloon 18 is slightly inflated to secure the stent 10 onto the exterior of the balloon. The catheter-stent assembly is introduced within the patient's vasculature in a conventional Seldinger technique through a guiding catheter (not shown). A guidewire 24 is disposed across a stenosed area or the damaged arterial section having a detached or dissected lining 22, and then the catheter-stent assembly is advanced over a guidewire 24 within the artery 20 until the stent 10 is directly under the detached lining 22. The balloon 18 of the catheter is expanded, expanding the stent 10 against the artery 20, which is illustrated in FIG. 2. While not shown in the drawing, the artery 20 may be expanded slightly by the expansion of the stent 10 to seat or otherwise fix the stent 10 to prevent movement. In some circumstances during the treatment of stenotic portions of an artery, the artery may have to be expanded in order to facilitate passage of blood or other fluid therethrough.

In a preferred embodiment, stent 10 serves to hold open the artery 20 after the catheter 12 is withdrawn, as illustrated by FIG. 3. The cylindrical elements 14 of stent 10 which are pressed into the wall of the artery 20 will eventually be covered with endothelial cell growth which further minimizes blood flow interference. The undulating portion of the cylindrical sections 14 provide good tacking characteristics to prevent stent movement within the artery. Furthermore, the closely spaced cylindrical elements 14 at regular intervals provide uniform support for the wall of the artery 20.

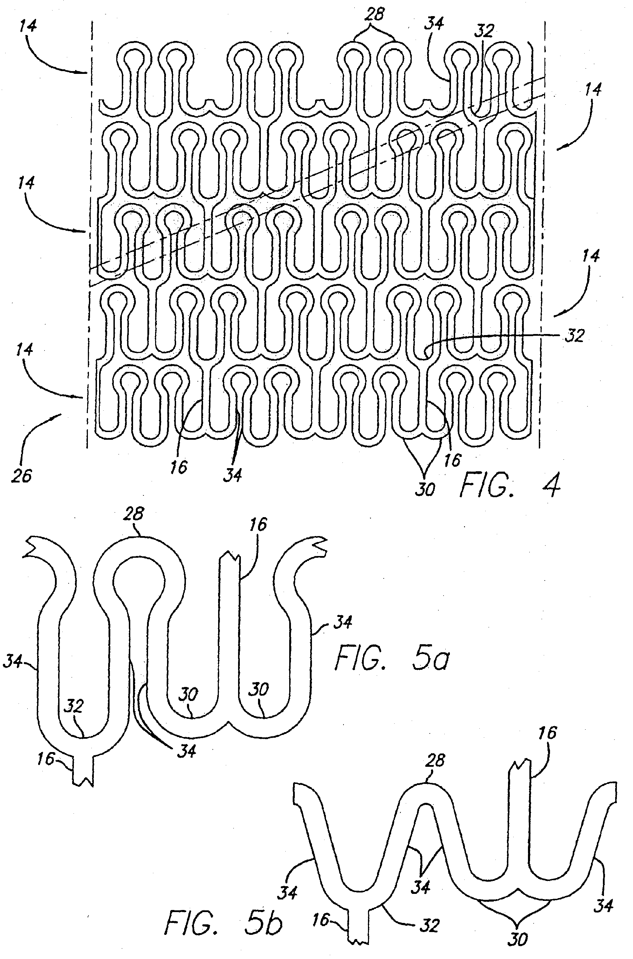

The stent pattern 26 from the stent 10 of the embodiment of FIGS. 1-3 is depicted in greater detail in FIG. 4. The stent pattern 26, which is depicted two-dimensionally as if the tubular stent were cut longitudinally and "unrolled" to form a flat sheet, includes a series of Us, Ws, and Ys that, in combination, define a stent 10 having a series of cylindrical elements 14 connected by links 16 disposed between adjacent elements.

FIGS. 5a and 5b depict a closeup view of a portion of the stent pattern 26 from FIG. 4. FIG. 5a depicts the portion as it would appear when the stent is in delivery but unbent configuration, while FIG. 5b depicts how parts of the stent flex or otherwise deform when the stent is placed in a bent condition. It can be seen that many portions of the stent have flexed or otherwise deformed as the stent is bent from the straight condition depicted in FIG. 5a to the bent/flexed condition of FIG. 5b. For example, the curved bases 28 of the Us, the curved bases 30 of the Ws, and the curved bases 32 of the Ys have changed shape. During delivery through a tortuous artery, these curved portions will bend and deform, depending on the bends of the particular artery and on the particular stent design, in order to permit the stent to bend. These bent or otherwise deformed portions, also known as flexing portions, are primarily responsible for stent flexibility. Note that the flexing and bending of the curved bases 28, 30, 32 depicted in FIG. 5b is partially exaggerated to more clearly depict the flexing of the structure that may occur as the stent is bent. Also, depending on the particular bends imposed on the stent, the flexing of the curved bases 28, 30, 32 may occur in varying degrees and in different directions, such inward (as opposed to the outward flexing depicted in FIG. 5a) and/or out of the "plane" of the stent surface.

While the flexing portions bend or otherwise deform, other portions do not appreciably flex during stent bending. For example, the links 16, as well as the bars 34 of the Us, Ws, and Ys, are still straight. Although the bars 34 may be in a slightly different position, they are not themselves bent. For example, the bars 34 were parallel to each other and to the longitudinal axis of the stent 10 prior to stent bending (as depicted in FIG. 5a), but after stent bending (depicted in FIG. 5b) are at angles from the longitudinal axis. This change in the bars' respective positions, however, is the result of deformation to the curved bases 28, 30, 32 of the Us, Ws, and Ys. The links 16 and bars 34 are themselves stable, in that they do not substantially bend or otherwise deform.

Because the links 16 and bars 34 of the stent pattern 26 depicted do not substantially bend or otherwise deform, they are not believed to make significant contribution to the flexibility of the stent. Accordingly, the links 16 and bars 34 could be made significantly stiffer without compromising overall stent flexibility.

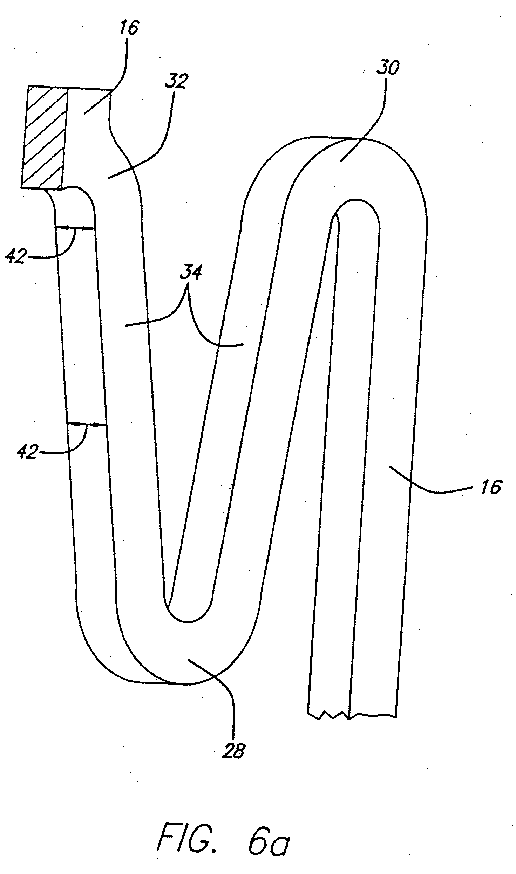

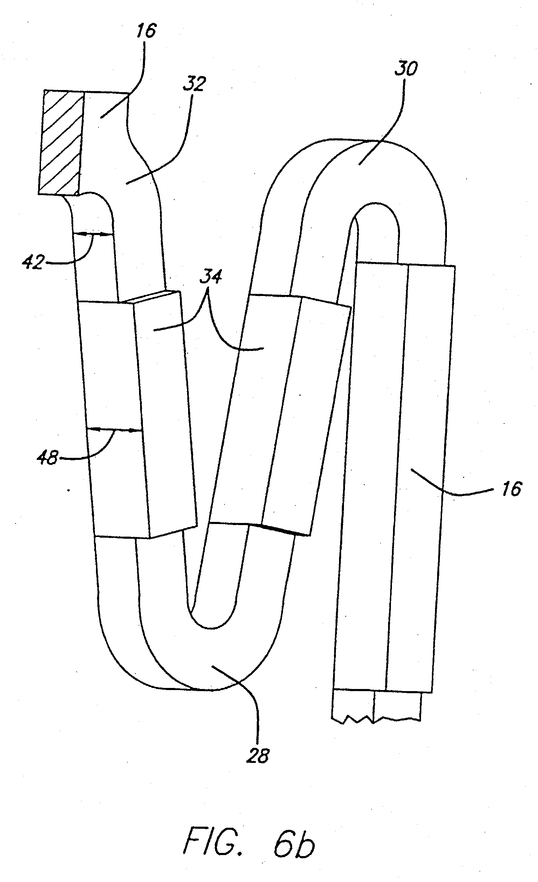

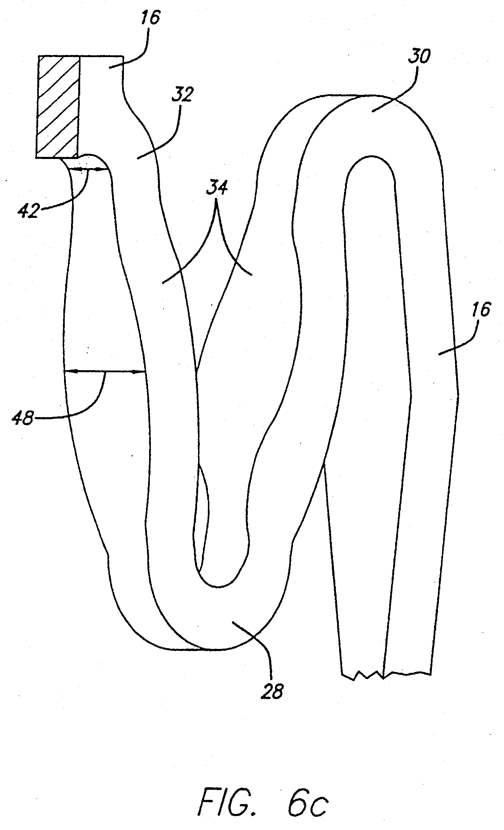

The current invention provides additional thickness to all or part of the stable portions of the stent. FIG. 6a depicts a perspective view, in partial cross-section, of a stent portion similar to that which was shown two-dimensionally in FIGS. 5a and 5b. In the view of FIG. 6a, it can be seen that all portions of the stent, i.e., the curved bases 28, 30, 32 as well as the links 16 and bars 34, have a nominal thickness 42. FIG. 6b depicts essentially the same design, except with varying thicknesses. In the embodiment of FIG. 6b, the flexing portions (i.e., the curved bases 28, 30, 32) of the stent have a nominal thickness 42, while the stable (i.e., largely non-flexing) links 16 and bars 34 have a greater-than-nominal thickness 48 without compromising overall stent flexibility. These thicker links 16 and bars 34 can provide additional strength and also provide enhanced radiopacity characteristics to the stent 10. FIG. 6c depicts a further embodiment, wherein the thickness smoothly transitions from a nominal thickness 42 in the curved bases 28, 20, 32 to a maximum greater-than-nominal thickness 48.

The preparation of a stent in accordance with the invention can be accomplished in a variety of ways. An initial step is to select a stent pattern and identify stable portions thereof. The identification of stable portions can be accomplished using many different techniques. For example, a computer-based modeling of the stent pattern can be performed that models the stent pattern during bending of the stent. A physical model or actual stent bearing the stent pattern can be prepared and subjected to bending, so that flexing and stable portions of the stent pattern can be identified by inspection of the physical device. Other approaches for identifying the flexing and stable portions are known in the art and are within the scope of the invention.

In the cited preferred embodiment, a stent is developed that optimizes flexibility while preserving radiopacity. Similar approaches could, however, be applied in developing a stent with enhanced deployment characteristics and good radiopacity. The above-cited approaches of providing a stent with thinner flexing portions and thicker stable portions, thus providing a stent with high flexibility and good radiopacity, could also be applied to develop a stent with good radiopacity and good expansion characteristics.

In the particular stent design depicted in FIGS. 5a and 5b, the flexing portions that promote stent flexibility coincide with portions that deform when the stent is expanded from its delivery configuration to its deployed configuration. By reducing the thickness of the portions that deform during stent deployment, greater uniformity of stent expansion may be achieved. Thus, in the particular embodiment depicted, reducing the thickness of the portions that flex during stent delivery also results in reduced thickness for the portions that flex during stent deployment, which may increase uniformity of stent expansion. For other stent designs, such as stents where the portions that flex during expansion do not coincide with the portions that flex during stent bending, the invention could be applied to increase uniformity of stent expansion. Such an approach would be similar to that discussed above, except that instead of (or in addition to) identifying portions of the stent that flex during stent bending, the methodology would involve identification of the portions of the stent that deform during stent expansion. By selectively providing such flexing portions with thicknesses that are less than the maximum thickness of the stent, improved stent deployment characteristics may be achieved.

It should be noted that, because the stable portions of the stent are by definition the portions of the stent that do not flex, identification of all flexing portions of a stent will inherently identify, through a process of elimination, the stable portions of the stent. Moreover, depending on the particular stent pattern and application, it may not be necessary to identify all stable portions. For example, for a stent where only a few stable portions will have greater-than-nominal thickness, it is only necessary to identify those few stable portions.

After identification of the stable portions has occurred, a stent is prepared having the desired stent pattern and thicknesses. Various techniques may be used to manufacture the stent. For example, a rough stent form can be prepared having a nominal thickness, and the stent pattern can be cut or otherwise created in the rough stent form. Additional material can be added to the stent surface on selected stable portions until the selected stable portions reach the desired greater-than-nominal thickness. Various techniques can be used to add the additional material, including sputter-coating, electroplating, or chemical vapor deposition.

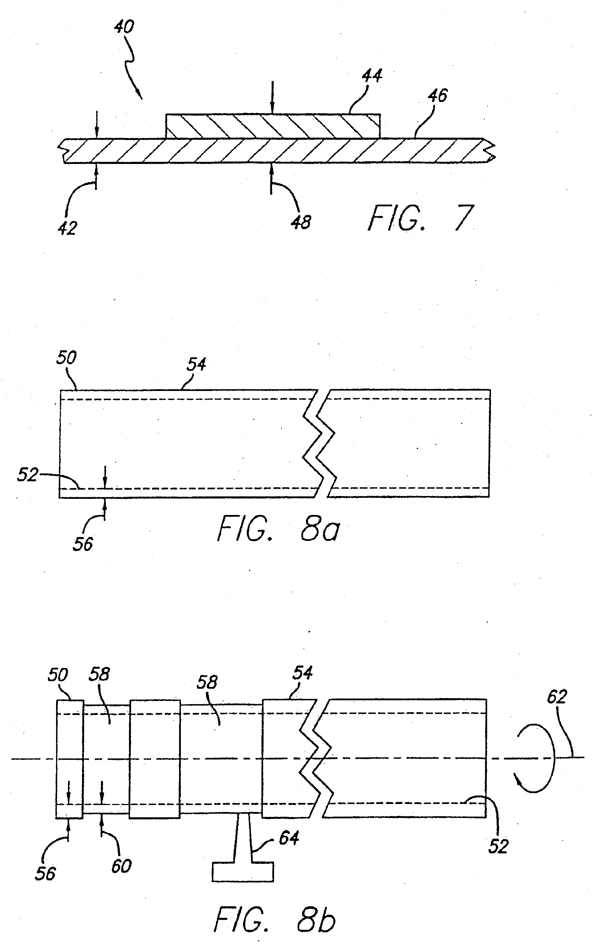

In the embodiment of Fig. 7, which depicts a close-up cross-section of a portion of a stent, the rough stent form 40 has a nominal thickness 42, and additional material 44 has been added to selected portions of the outer stent form surface 46 to increase the thickness of the selected portions to a larger-than-nominal thickness 48. The above-cited steps could be performed in a different order, for example with the additional material added to the rough stent form prior to or even concurrently with the step of creating the stent pattern in the rough stent form.

In a preferred embodiment, the extra material added to thicken portions of the stent is the same material from which the rest of the stent was initially made. For example, in a stent initially formed of stainless steel with a nominal thickness, it is preferable that stainless steel be added to the selected stable portions of the stent to increase the thickness. By using the same material, the stent is a generally homogenous structure.

Due to manufacturing and other considerations, it may be desirable to start with a rough stent form having a desired larger-than-nominal thickness, and then selectively reducing the thickness of desired portions, such as the flexing portions, to a nominal thickness. In the embodiment depicted in FIG. 8a, a rough tubular section 50 is depicted having a greater-than-nominal thickness 56. Material is removed from selected portions 58 of the outer surface 54 of rough tubular stent form 50, which in the embodiment depicted in FIG. 8b involves machining, until a nominal thickness 60 is achieved. In the embodiment of FIG. 8b, the machining involves rotating the rough tubular section 50 about its longitudinal axis 62, such as may be accomplished using a lathe or other rotating device, and pressing a machining tool 64 against outer surface 54. Other techniques can also be used within the scope of the invention to remove the material, including chemical etching, laser ablation, centerless grinding, and/or milling.

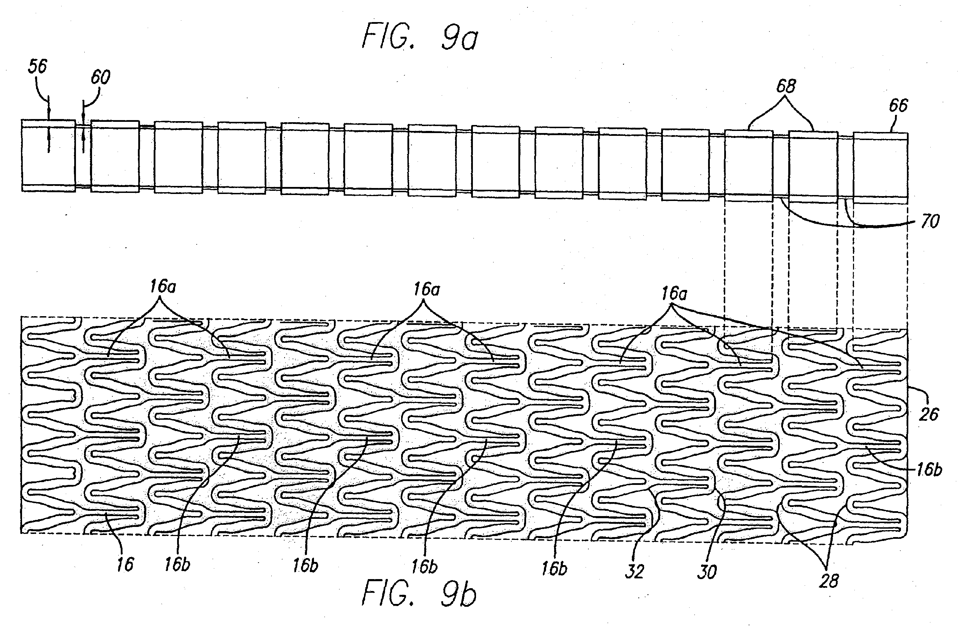

The result is a variable-thickness tubular section 66 having thicker areas 68 of the original greater-than-nominal thickness and thinner areas 70 with nominal thickness, as depicted in FIG. 9a. Next, the stent pattern 26 is cut into the tubular section 66. The stent pattern 26 of the particular embodiment is depicted in two-dimensional form in FIG. 9b, which is aligned with the tubular section of FIG. 9a to show that the flexing portions (which in this embodiment are the curved portions 28,30, 32) of the pattern 26 will overlie, when the pattern 26 is cut into the tubular section 66, the thinner areas 70 having nominal thickness. Accordingly, in the completed stent the flexing portions have a nominal thickness, while at least a part of the stable portions have a greater-than-nominal thickness.

The above-cited steps might be conducted in a different order. For example, a stent pattern could be cut into a rough tube prior to the step of reducing the thickness of the flexible portions.

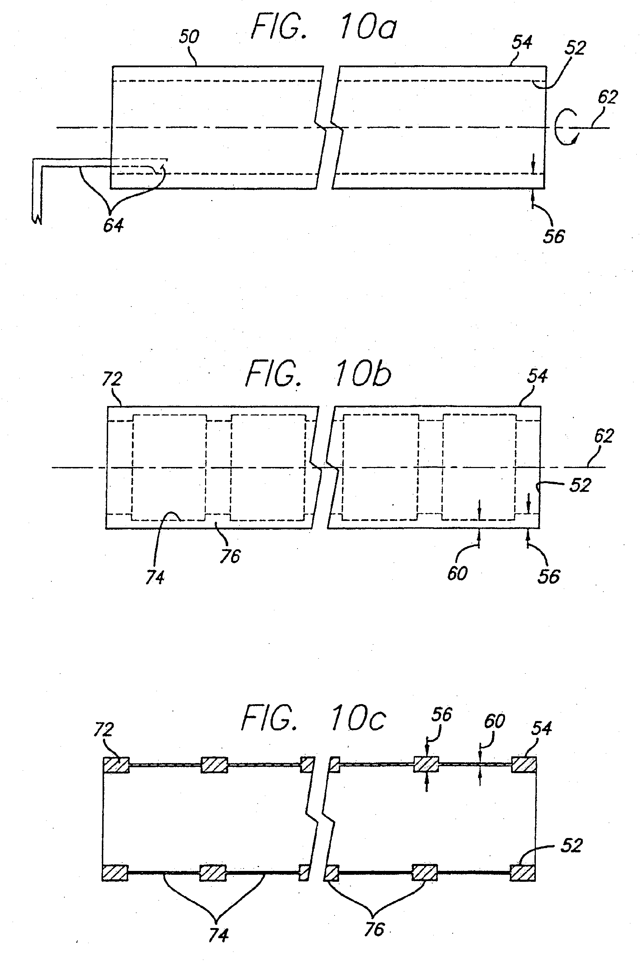

In another approach, the thinning of the selected portions may be accomplished from the inside of the rough tubular form. For example, as depicted in FIG.10a, the rough tubular stent form 50 has a greater-than-nominal thickness 56. A machining tool 64 is positioned inside the tubular rough stent form 50 and abutting the interior surface 52 of the rough stent form 50 as the rough stent form is rotated about its longitudinal axis 62. The resulting variable-thickness tubular structure 72, depicted in FIG. 10b, has thinner areas 74 with nominal thickness 60 and thicker areas 76 with the original greater-than-nominal thickness 56. The tubular structure 72 has a smooth outer surface 54, while the interior surface 52 shows the effects of the thinning process. Note that, depending on a particular application, the thinning could be performed on both the outside surface and inside surface of a tubular structure, as depicted in FIG. 10c.

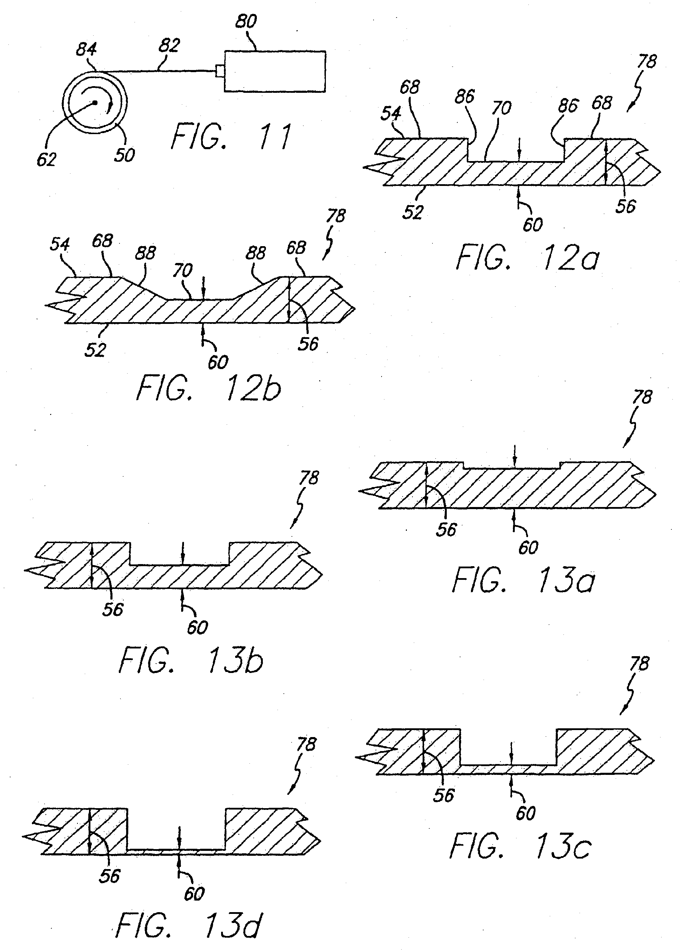

In addition to the physical machining methods outlined above, the reduction in thickness can be achieved through a variety of other methods, including ablating the selected surface areas. The ablation could be performed through various methods, including chemical and/or laser ablation. This step may also be performed as part of the process of cutting the stent pattern into the rough stent form. For example, where laser cutting is used to cut the stent pattern, the laser might also be used to thin desired portions of the rough stent form. Such thinning using a laser might involve changing the focus depth of the laser, changing the laser power, or using the laser to tangentially "shave" across the surface of the rough stent form, as depicted in FIG. 11. In FIG. 11, a laser 80 fires a beam 82 that tangentially strikes the surface 84 of a rough tubular form 50, thereby removing a layer of material. When the rough tubular form 50 is moved relative to the laser beam 82, such as by rotating about an axis 62 as shown, desired areas of thinner material can be achieved. Other movements of the stent relative to the laser beam could also be used, depending on the desired pattern. For example, the stent could be moved in any direction and/or rotated in any direction relative to the laser beam in order to achieve a desired result. Note that the stent itself does not have to physically moved if the laser beam is instead moved to cause a net result of stent movement relative to the laser beam.

It may be desirable to provide gradual transitions between areas of nominal and greater-than-nominal thickness. For example, FIG. 12a shows a close-up, in cross-section, of a portion of the wall section 78 of the rough tubular form 50 from the embodiment of FIGS. 8b and 9a. As depicted in FIG. 12a, the outer surface 54 has borders 86 that are well-defined between the thinner areas 70 of nominal thickness 60 and the thicker areas 68 of greater-than-nominal thickness 56. In another embodiment depicted in FIG. 12b, the outer surface 54 has a more gradual transition region 88 between the thinner areas 70 and the thicker areas 68. Thus, the outer surface 54 of the rough tubular form 50, and the resulting finished stent, is free of sharp drop-offs between adjacent areas, and instead has gradual ramping transition regions 88 that provide a smoother transition between adjacent areas. Such gradual transitions between adjacent areas can also be employed on the inner surface of the stent when the variations in thickness are present on the inner surface 52 as depicted in FIG. 10b. The gradual transition regions do not have to be straight ramps, but can be formed in a variety of simple and/or complex geometrical shapes, depending on the particular application.

The difference between the nominal thickness and the greater-than-nominal thickness can vary depending on the particular application, stent design, and on the material from which the stent is formed. For example, for some materials, a small difference in thickness can make a large difference in radiopacity. For other materials, a larger difference in thickness may be required.

In the embodiment depicted in FIG. 13a, the nominal thickness is about 80% (i.e., four-fifths) of the greater-than-nominal thickness. Other thickness ratios are also within the scope of the invention. For example, in the embodiment of FIGS. 13b, the nominal thickness is 50% of the greater-than-nominal thickness. In FIG. 13c, the nominal thickness is 25%, while in FIG. 13d the nominal thickness is down to 10% of the greater-than-nominal thickness. The nominal thickness could be even smaller or larger than the amounts set forth in FIGS. 13a to 13c, depending on the particular application and stent material. In general, the nominal thickness should be of a size that meets flexibility and structural requirements, while the greater-than-nominal thickness should be sufficient to meet radiopacity requirements. The thicknesses on a particular stent are application specific, and generally account for the specific material involved. For example, one embodiment of a coronary stent formed from stainless steel could have a nominal thickness of 0.0025 inch with a greater-than-nominal thickness of 0.0055 inch. A peripheral stent formed from a material with low radiopacity could have a nominal thickness of 0.003 inch and a greater-than-nominal thickness of 0.010 inch. A stent formed from highly radiopaque material might have a nominal thickness of 0.0025 inch and a greater-than-nominal thickness of 0.0035 inch.

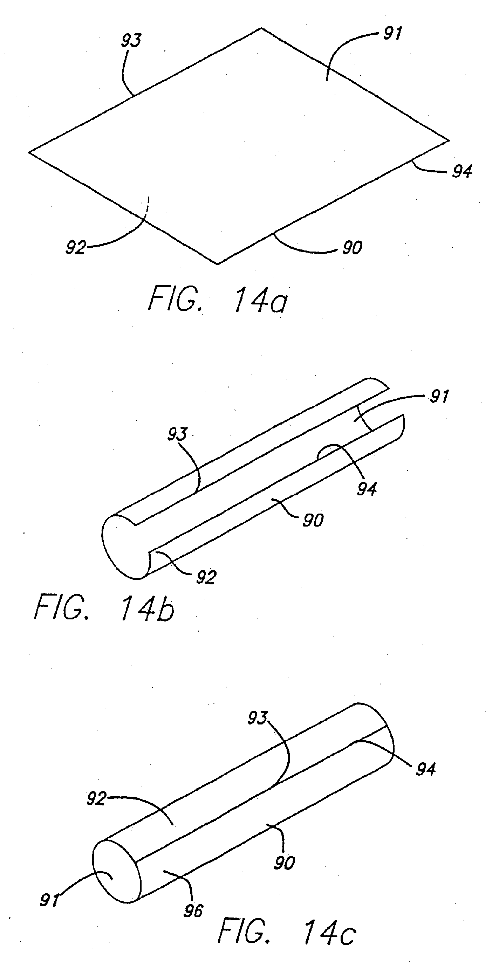

The rough stent form may be in the form of a tubular member, as discussed above and depicted in FIGS. 8a, 8b, 9a, and 9b. Other manufacturing techniques, however, can also be employed to manufacture a stent within the scope of the invention. One such approach starts with a rough stent form comprising a generally planar member 90 having a first side 91 and a second side 92, as depicted in FIG. 14a. Opposing ends 93, 94 of the planar member 90 are brought toward one another, as depicted in FIG. 14b, until they contact one another, depicted in FIG. 14c, so that the planar member 90 forms a tubular shape 96 with the first side 91 forming the inner surface and the second side 92 forming the outer surface. The opposing ends 93, 94, which are now adjacent, can be secured to one another through various methods, such as spotwelding or adhesives.

The above approach can permit the step of varying the thickness to be performed on the generally planar member 90 after it is rolled into a tubular shape. Such steps could be performed in various ways, including the techniques set forth above with respect to the tubular member depicted in FIGS. 8a, 8b, 9a, and 9b. The approach of starting with a generally planar member 90 can also permit the step of varying the thickness to be performed prior to the step of rolling the planar member 90 into a tubular shape 96. For example, material could be selectively thinned from, or added to, the first side 91 of the planar member 90 to vary the thickness of the planar member 90. Thus, when the planar member 90 is rolled into the tubular shape 96, the first side 91, which is the inner surface of the tubular shape 96, will have an uneven surface caused by the differences in thickness. Another approach is to have material selectively thinned from, or added to, the second side 92 prior to rolling of the planar member 90. Such an approach would result in the outer surface of the tubular member 96, which is the second side 92, having an uneven surface caused by the differences in thickness. The approaches could be combined, so that material is added to and/or removed from both the first side 91 and the second side 92, resulting in a uneven surfaces on both the first side 91 and the second side 92. After rolling of the planar member 90, the resulting tubular shape 96 would thus have an uneven inner surface and an uneven outer surface.

The approach of starting with a generally planar member 90 can also permit the step of cutting of the stent pattern to be performed prior to or after the planar member 90 is rolled into the tubular shape 96.

Another approach involves forming the stent from one or more sections of wire, which can involve bending or otherwise forming the wire into the desired stent pattern. The forming of the wire into the desired stent pattern may involve spot-welding or other methods of connecting the wire. The wire may be initially formed with varying thickness, which may be accomplished using various techniques such as adjustable die extrusion. The modification in thickness could occur after the wire is formed. Depending on a particular application, the modification in thickness could be performed prior to, after, or even during the process of bending or otherwise forming the wire into the desired stent pattern. Such modifications in thickness could involve adding and/or removing material from the wire, and could be performed using a variety of techniques, including necking of the wire, machining, sputter coating, and/or plating.

FIGS. 23a-c depict an example of forming such a stent from wire. In FIG. 23a, wire 170 is pulled from a spool 172 and then through a necking mechanism 174. In a motion timed to correspond to desired lengths of wire, the necking mechanism 174 selectively contacts the wire 170 as the wire is drawn therethrough, thus creating narrow portions 176 on the wire 170. The resulting wire shape is depicted in close-up in FIG. 23b, with the narrow portions 176 having a reduced thickness 175 compared to the larger thickness 177 of the rest of the wire 170. The size, spacing, and other characteristics of the wire and its narrow portions are selected in accordance with a desired stent pattern. As depicted in FIG. 23c, the wire 170 is bent or otherwise deformed into a desired stent pattern 178 (of which only a portion is depicted in FIG. 23c). The stent pattern 178 may include securing points 179 where adjacent wire portions are spot-welded or otherwise secured together. Note that the thin portions 176 of the wire 170 are positioned so as to correspond to curved portions 180 of the stent pattern 178, which in a particular embodiment can coincide with flexing portions of the stent pattern. The resulting stent has flexing portions that have a lesser thickness than the stable portions. For different stent patterns and/or different applications, wires with different characteristics can be used, such as wires having thin portions with different spacing therebetween as well as variations in other characteristics.

Other manufacturing methods and techniques are also within the scope of the invention, as long as the resulting stent has a nominal thickness one or more flexing portions and a greater-than-nominal thickness on one or more stable portions.

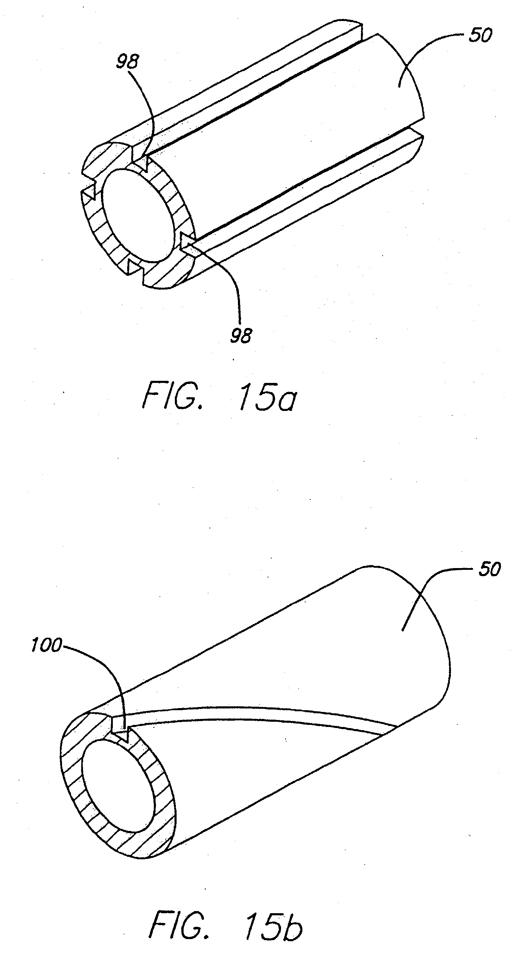

FIGS. 8a through 10b depict thinner and thicker portions that extend radially about a tubular stent, as could be achieved by machining a tubular structure using a lathe. The thin and thick portions could, however, be spread across the stent in a variety of patterns, depending on the particular stent design and the desired characteristics. For example, the thick and thin portions can extend longitudinally along the stent length. An embodiment of such an approach is depicted in FIG. 15a, which depicts three thinner areas 98 extending longitudinally down a rough stent form 50. The thin and thick portions might extend in helical patterns that wind about the stent. FIG. 15b depicts an example of such an embodiment, wherein a thinner area 100 helically winds around the rough stent form 50. Such patterns could be accomplished through a variety of techniques, such as helical interpolation. Various patterns could be combined, such as having a stent with longitudinally and radially extending portions of thinner areas.

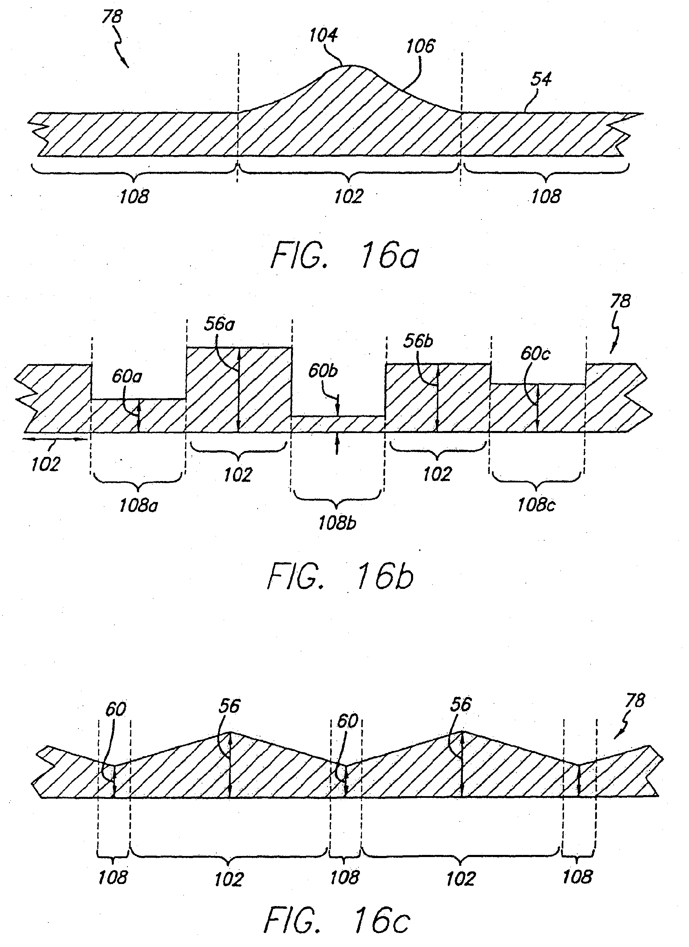

Not all stable portions of the stent need to have the same greater-than-nominal thickness. For example, in the embodiment of FIG. 16a, a stent section 78 has stable portions 102 with varying thicknesses that gradually increase toward the centers 104 of the stable portions. Such variations in thickness can provide varying radiopacity, as well as create a "smoother" outer surface 54 having gradual slopes 106 instead of sharp rises or falls.

Not all flexing areas of the stent need to have the same nominal thickness. Flexing areas could have gradual slopes, such as depicted for the stable portions 102 in FIG. 16a. As another example, the embodiment depicted in FIG. 16b shows different flexing areas 108a, 108b, 108c having different thicknesses 60a, 60b, 60c. In the particular example, the thicknesses 56a, 56b of some stable portions 102 also vary. The thickness can be selected depending on the desired flexing of a particular area. Although the thicknesses 60a, 60b, 60c vary, they are all thinner than the thickest of the stable portions 56a and, in the particular embodiment depicted, are also thinner than even the thinnest of the stable portions 56b shown. The thickness of the stable and flexing portions could continually vary along the stent, as depicted in FIG. 16c. The thickness of the embodiment of FIG. 16c ramps up and down from a minimum thickness 60 in the flexing portions 108 to a maximum thickness 56 in the stable portions 102. While FIG. 16c depicts a simple ramping transition to vary the thickness along the stent, other geometric patterns could also be used, such as sinusoidal wave-like structures.

Note that some of the stable portions could be thinner than the flexing portions. Such thin stable portions might, however, be difficult to see with a fluoroscope, depending on the radiopacity of the particular material. In addition to such thin stable portions, other thicker stable portions could be provided that have the necessary thickness for radiopacity. If properly located on the stent, with such locations dependent on the particular application, these thicker stable portions could be adequate to meet overall radiopacity requirements in order to permit a user to easily see the position of the stent with a fluoroscope.

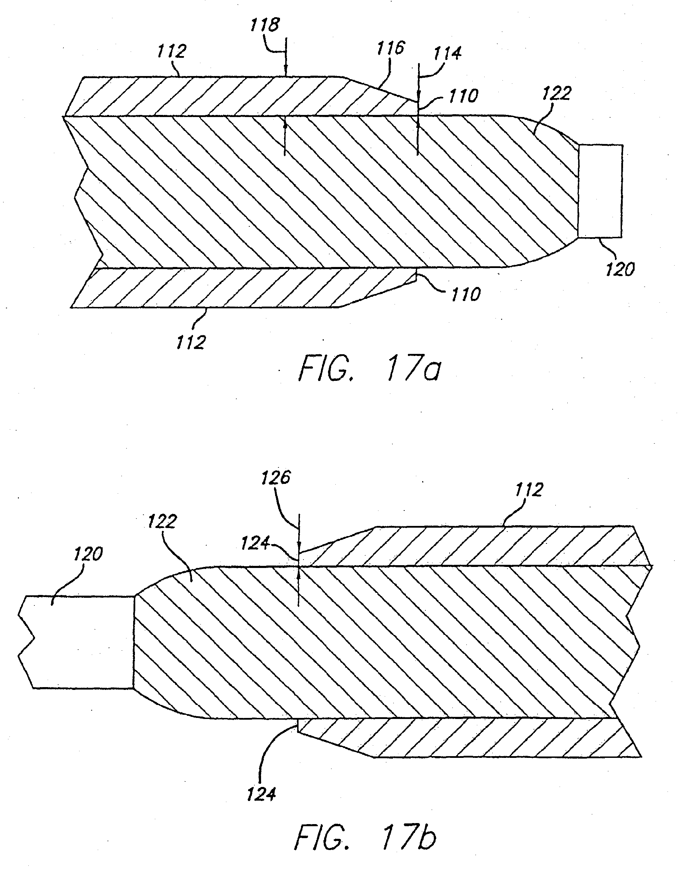

The outer surface of the stent can be tailored to meet particular requirements. For example, in the embodiment of FIG. 17a, the distal edge 110 of a stent 112 is provided with relatively small thickness 114, with a gradually sloping area 116 that grows to a larger thickness 118. The thin distal edge 110 provides a low delivery profile for the stent 112. When mounted on a catheter 120 with a balloon 122, or on another delivery device, the thin distal edge 110 gives the overall balloon/stent structure a minimal distal profile, which can enhance deliverability and deployment of the stent 112 by helping to avoid vessel trauma and allowing the device to cross tight lesions. A similar technique can be applied to the proximal edge 124 of the stent 112, depicted in FIG. 17b, which has a relatively small thickness 126 that reduces the proximal profile of the overall balloon/stent structure. Such a thin proximal edge 124 can enhance the ability of the balloon/stent structure to be withdrawn if the user so desires.

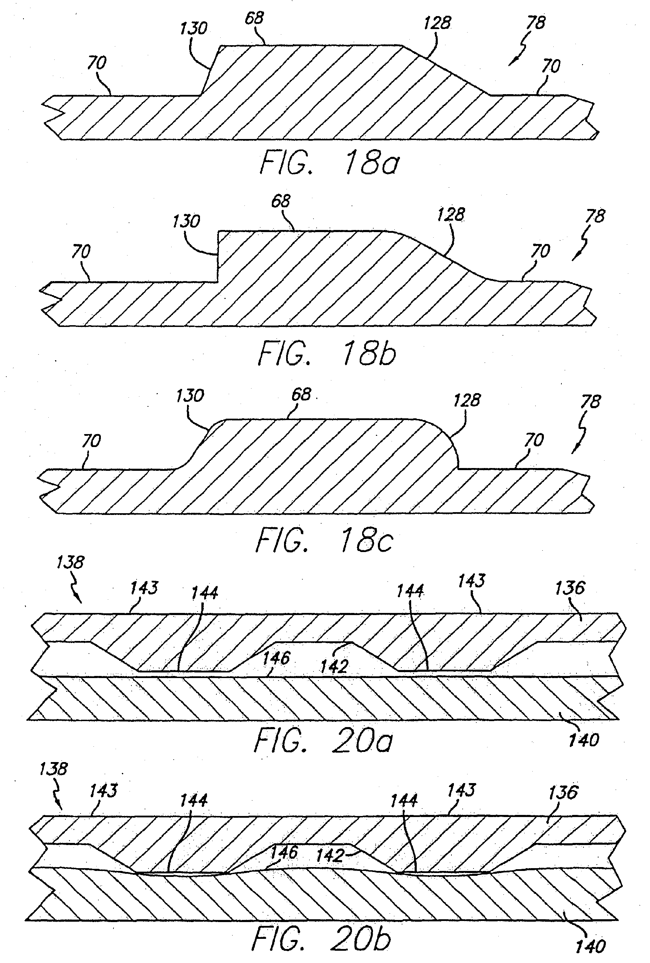

The use of gradual sloping areas can be used in other portions of the stent surface to enhance deliverability. For example, in the embodiment depicted in FIG. 18a, areas of greater thickness 68 have distal transition areas 128 that are gradually sloped, which may make the stent easier to (distally) advance. The proximal transition areas 130 are not so gradually sloped. Such gradual slopes and less gradual slopes could be combined in a variety of manners to the proximal and distal transition areas, such as are depicted in FIGS. 18b and 18c. As stated previously, the sloping of such areas can range from simple sharp cutoffs to any number of complex geometric patterns. Moreover, the invention is not limited to any particular geometry at any particular site on the stent. Various geometries can be used on a single stent. The geometries can vary across particular sites on the stent to enhance desired characteristics at the particular sites. Various geometries could have potentially therapeutic benefits in avoiding vessel trauma during stent delivery and/or deployment, as well as engineering benefits such as improved fatigue and strength properties from using the smooth transitions.

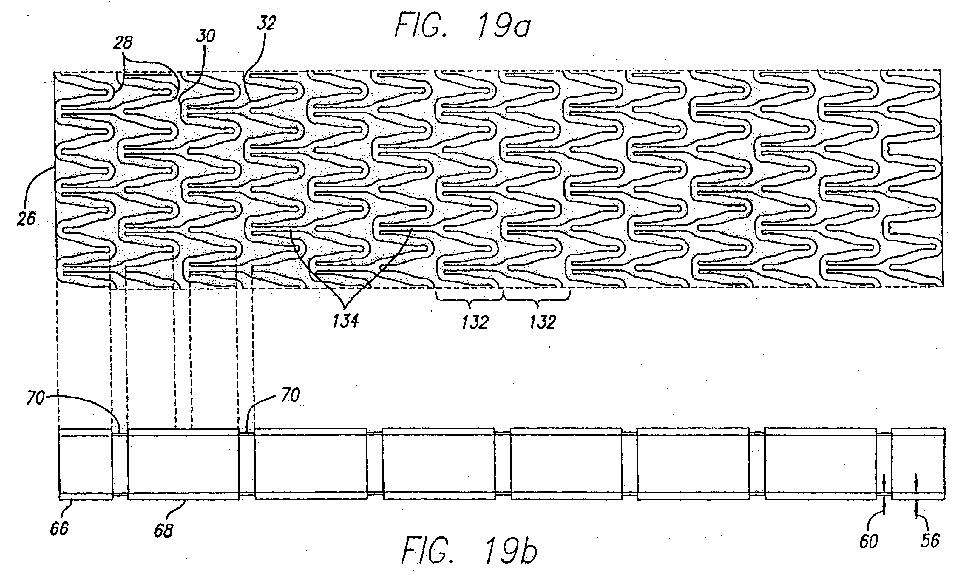

In a further embodiment, not all flexing portions of the stent correspond to thinner areas. In the example depicted in FIG. 19a, which depicts the stent pattern from FIG. 9a, flexing portions of the stent pattern 26 coincide with the curved bases 28, 30, 32 of the Us, Ws and Ys. Thinner areas 70 of the rough stent form, depicted in FIG. 9b, only coincide with about half of the flexing portions passing along the length of the stent. In the particular embodiment, the stent comprises convoluted rings 132 connected by links 134, with the curved portions 28, 30, 32 that lie at the distal and proximal areas of each ring 132 corresponding to the flexing portions. In other words, instead of thinning the distal and proximal areas of each ring 132, every other region of flexing portions has been skipped. Such an approach may not maximize overall flexibility, but could be used to enhance local flexibility at selected locations on the stent. Depending on the particular application, the approach could be used to tailor local flexibility of select portions of the stent while maintaining other desired characteristics such as radiopacity, strength, etc.

Variable thickness can provide an improved method of retaining a stent onto a balloon. For example, in the embodiment of FIG. 20a, a portion 136 of a stent 138 is shown positioned on a balloon 140. The stent inner surface 142 has a varying structure, similar to the embodiments depicted in FIG. 10b, where variable thickness is achieved by varying the surface structure of the inner surface 142. FIG. 20a depicts the stent 136 loosely positioned on the balloon 140. FIG. 20b depicts the same stent portion 136 after the stent 138 has been crimped onto the balloon 140. As shown in FIG. 20b, the thicker portions 143 of the stent 138 created inner surface "projections" 144 that, after the stent 138 is crimped onto the balloon 140, press against the balloon surface 146 and help to secure the stent 138 to the balloon 140.

FIGS. 21a and 21b are similar to FIGS. 20a and 20b, except that the stent 138 of FIGS. 21 a has, prior to the stent 138 being crimped onto the balloon 140, a relatively smooth inner surface 142 but an uneven outer surface 148. When the stent 138 is crimped onto the balloon 140, as depicted in FIG. 21b, the higher portions or peaks 150 of the outer surface 148 are subject to greater pressures than the lower portions or valleys 152 of the outer surface. Because the stent 138 is formed from relatively thin material, this greater pressure causes the inner surface portions 154 opposite the outer surface peaks 150 to deform inwardly respective to the rest of the inner surface 142. Accordingly, although the uncrimped stent depicted in FIG. 21 a had a relatively smooth inner surface, the crimped stent in FIG. 21b has inwardly projecting portions 156 that serve to retain the stent 138 onto the balloon 140.

The embodiments of FIGS 20a and 21a could be combined, with a stent having uneven outer and inner surfaces prior to be crimped onto a balloon. If the inner surface projections 144 from the embodiment of FIG. 20a were aligned with the outer surface peaks 150 from FIG. 21a, inward deformation of the outer surface peaks would cause a corresponding deformation of the inner surface projections toward the center of the stent. This increase in the inner surface projection deformation could help to secure the stent onto the balloon.

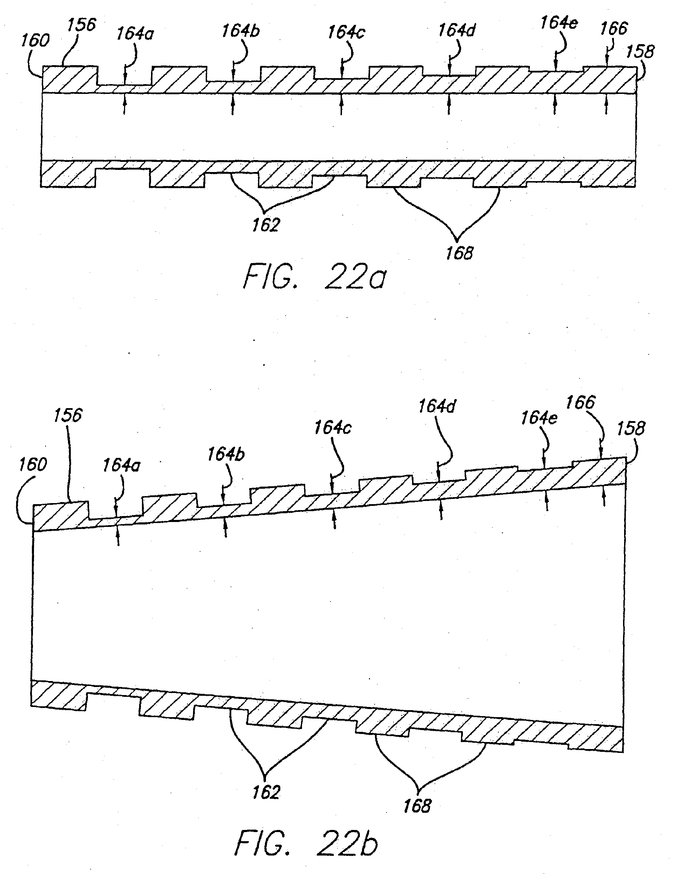

As discussed above, varying the thickness of flexible portions across the stent could be used to tailor local flexibility of stent portions. Similarly, by applying varying thickness across the stent to stent portions that deform during stent expansion, deployment characteristics of the local portions of the stent can tailored to suit desired requirements. For example, a stent having thinner portions may, depending on the location of the thin portions, have greater recoil than a thicker stent. By combining thinner and thicker regions across a single stent, a stent can be designed having different recoil characteristics across its structure. FIG. 22a depicts an embodiment of such a stent 156 having a distal end 158 and a proximal end 160. So-called "deforming" areas 162 of the stent 156 (i.e., those areas that deform during stent expansion) are provided with varying thicknesses 164a-e. Toward the distal end 158 of the stent 156, the deforming areas 162 have a distal thickness 164e approaching that of the thickness 166 of the stable areas 168. The thicknesses 164a-e of the deforming portions gradually decreases going toward the proximal end 160, finally reaching a proximal thickness 164a. The proximal thickness 164a is relatively small, thereby increasing the recoil at the stent proximal end 160. Thus, the stent 156 at its proximal end 160 will recoil to a greater degree than the distal end 158. In the specific embodiment shown, the design results in a stent that can have a tapered shape, as depicted in FIG. 22b, after expansion. Note that such an approach is not limited to tapered stents. By varying the thickness of deforming portions across the stent, deployment characteristics can be tailored at various locations across the stent. Stents can thus be designed having any number of deployment characteristics, including various shapes that can be lesion-specific. The approach could also be used to create a stent having varying radial strength from end to end.

Note that FIGS. 22a and 22b are for illustrative purposes only, as the figures do not illustrate all details of a stent. For example, the stent pattern is not depicted in those figures.

It is not necessary to have greater-than-nominal thickness on all stable portions of the stent. Depending on a particular application, only a very small number of the stable portions need to have the extra thickness for radiopacity. Those stable portions with the extra thickness for radiopacity could be positioned along the length of the stent, so that the stent is visible for essentially its entire length. For example, in a stent having the pattern depicted in FIG. 9b, the extra thickness could be provided on just the connecting links 16, or even on just a few of these links, such as the links specifically identified on the figure with the reference number 16a. In another embodiment, even fewer of the links, such as those specifically identified with reference number 16b, could be provided with the extra thickness, depending on the particular application. Such embodiments would, however, require different thickness ratios than is depicted in FIG. 9a. As another approach, a stent could be designed wherein only the stable portions near the proximal end and distal ends of the stent had the greater-than-nominal thickness. This would provide enhanced radiopacity for the end portions of the stent, so that a user could easily see where the stent begins and ends, thus aiding in positioning of the stent.

The invention can be combined with other stent design approaches. For example, the varying thicknesses of this invention could be combined with a stent pattern having varying widths of stent struts, links, and other stent portions.

Although preferred and alternative embodiments of the invention have been described and illustrated, the invention is susceptible to modifications and adaptations within the ability of those skilled in the art and without the exercise of inventive faculty. Thus, it should be understood that various changes in form, detail, and usage of the present invention may be made without departing from the spirit and scope of the invention. For example, while the present invention has been described herein in terms of an expandable stent for use within a patient's blood vessel, the invention can also be employed for stents for use within other body lumens. Accordingly, it is not intended that the invention be limited, except as by the appended claims.

## Claims

1. A method of manufacturing a stent with variable thickness, comprising the steps of:
providing a generally tubular section with a desired thickness;
varying the thickness of selected portions of the tubular section; and
removing material from the tubular section to form a desired stent pattern.

2. The method of claim 1, wherein the step of removing material includes the step of laser cutting the desired pattern in the tubular section.

3. The method of claim 1, wherein the step of varying the thickness of selected portions of the tubular member includes the steps of:
mounting the tubular member on a rotating mechanism;
rotating the tubular member;
pressing a machining tool against a portion of the tubular member until the thickness of the tubular member portion is reduced to a nominal thickness.

4. The method of claim 3, wherein the tubular section has an inner surface, and wherein the step of pressing the machining tool against the tubular member includes pressing the machining tool against the inner surface of the tubular member.

5. The method of claim 3, wherein the tubular section has an outer surface, and wherein the step of pressing the machining tool against the tubular member includes pressing the machining tool against the outer surface of the tubular member.

6. A method of manufacturing a stent with variable thickness, comprising the steps of:
providing a generally planar section with a desired thickness, the generally planar section having a first surface and a second surface;
varying the thickness of one or more selected portions of the generally planar section;
removing material from the generally planar section to form a desired stent pattern; and
rolling the generally planar section into a tubular shape such that the first surface forms an inner surface of the tubular shape and the second surface forms an outer surface of the tubular shape.

7. The method of claim 6, wherein the step of rolling the generally planar section into a tubular shape occurs after the step of removing material from the generally planar section to form the desired stent pattern.

8. The method of claim 6, wherein the step of removing material to form the desired stent pattern includes the step of laser cutting the desired stent pattern in the generally planar section.

9. The method of claim 6, wherein the step of varying the thickness of selected portions of the generally planar member occurs after the generally planar member has been rolled into a tubular shape, and wherein the step of varying the thickness includes the steps of:
mounting the tubular shape on a rotating mechanism;
rotating the tubular shape;
pressing a machining tool against a portion of the tubular shape until the thickness of the tubular shape portion is reduced to a nominal thickness.

10. The method of claim 9, wherein the step of pressing the machining tool against the tubular shape includes pressing the machining tool against the first surface of the tubular shape.

11. The method of claim 9, wherein the step of pressing the machining tool against the tubular shape includes pressing the machining tool against the second surface of the tubular shape.

12. A method of manufacturing a stent with variable thickness, comprising the steps of:
selecting a stent pattern;
determining flexing portions and stable portions of the stent pattern;
forming a stent with the desired stent pattern, including the steps of:
providing one or more flexing portions of the stent pattern with a nominal thickness; and
providing one or more stable portions of the stent pattern with a greater-than-nominal thickness.

13. The method of claim 12, wherein the step of providing flexing portions of the stent pattern with a nominal thickness includes the step of reducing the thickness of the flexing portions of the stent from a greater-than-nominal thickness to a nominal thickness.

14. The method of claim 13, wherein the step of reducing the thickness of the flexing portions of the stent from a greater-than-nominal thickness to a nominal thickness includes the step of removing a surface layer of material from the outer surface of the stent.

15. The method of claim 13, wherein the step of reducing the thickness of the flexing portions of the stent from a greater-than-nominal thickness to a nominal thickness includes the step of removing a surface layer of material from the inner surface of the stent.

16. The method of claim 12, wherein the stent is formed from a desired material, and the step of providing one or more stable portions of the stent pattern with a greater-than-nominal thickness includes the step of increasing the thickness of one or more stable portions of the stent from a nominal thickness to a greater-than-nominal thickness by adding additional amounts of the desired material to the outer surface of the stent.

17. The method of claim 16, wherein the step of increasing the thickness of one or more stable portions of the stent includes sputter-coating additional amounts of the select material onto the outer surface of the stent.

18. The method of claim 12, wherein the step of forming the stent with the desired stent pattern includes the step of forming a wire into the desired stent pattern.

19. The method of claim 18, wherein the step of forming the wire into the desired stent pattern includes the step of forming the wire with variable thickness.

20. The method of claim 12, wherein the step of forming a stent with the desired stent pattern includes the steps of:
providing a rough stent form; and
cutting the desired stent pattern into the rough stent form.

21. The method of claim 20, wherein the step of reducing the thickness of one or more flexing portions of the stent from a greater than nominal thickness to a nominal thickness includes the step of removing a surface layer of material from the outer surface of the stent.

22. The method of claim 21, wherein the step of removing a surface layer of material from the outer surface of the stent is performed prior to the step of cutting the desired stent pattern into the tubular member.

23. A stent for deployment in a body lumen, the stent comprising:
a generally tubular member having a desired stent pattern, the desired stent pattern comprising:
one or more flexing portions, wherein at least some of the flexing portions have a nominal thickness, and
one or more stable portions, wherein at least some of the stable portions have a greater-than-nominal thickness.

24. The stent of claim 23, wherein the nominal thickness is 10% or less of the greater-than-nominal thickness.

25. The stent of claim 23, wherein the nominal thickness is 50% or less of the greater-than-nominal thickness.

26. The stent of claim 23, wherein the nominal thickness is 80% or less of the greater-than-nominal thickness.

27. The stent of claim 23, wherein the flexing portions and stable portions are formed from the same material.

28. The stent of claim 23, wherein the stent has a relatively uneven outer surface.

29. The stent of claim 23, wherein the stent has a relatively uneven inner surface.

30. The stent of claim 29, wherein the stent has a relatively uneven outer surface.

31. A method of securing a stent to a balloon, comprising the steps of:
providing a stent with projections on an inner surface thereof;
positioning the stent onto an outer surface of the balloon;
crimping the stent onto the balloon, whereby the projections on the stent inner surface engage the outer surface of the balloon.

32. The method of claim 31, including, prior to the step of crimping the stent onto the balloon, the further step of:
providing outer projections on an outer surface of the stent, said outer projections and stent configured so that inward pressure against the outer projections will deform an inner surface of the stent in such a way as to increase the inner projections on said the stent inner surface.

33. A method of securing a stent to a balloon, comprising the steps of:
providing a stent with outer projections on an outer surface thereof, said outer projections and stent configured so that inward pressure against the outer projections will deform an inner surface of the stent in such a way as to create inner projections on said the stent inner surface;
positioning the stent onto an outer surface of the balloon;
crimping the stent onto the balloon, wherein the step of crimping includes the step applying inward pressure against the outer surface projections, thereby creating inner projections on the inner surface of the stent that engage the outer surface of the balloon.

34. A stent for deployment in a body lumen, the stent comprising:
a generally tubular member having a desired stent pattern, said desired stent pattern comprising:
one or more deforming portions that will deform when the stent is expanded from a delivery condition to a deployed condition, wherein at least some of the deforming portions have a nominal thickness, and
one or more non-deforming portions that will not substantially deform when the stent is expanded from a delivery condition to a deployed condition, wherein at least some of the non-deforming portions have a greater-than-nominal thickness.
